# EUROPEAN PATENT SPECIFICATION

(11) **EP 4 346 647 B1**
(45) Date of publication and mention of the grant of the patent: **22.04.2026**
(21) Application number: 22740574.3
(22) Date of filing: 01.06.2022
(51) Int. Cl.: A61B 17/22, A61F 2/24, A61B 17/00

(54) **KIT FOR USE IN DELIVERING A REPLACEMENT HEART VALVE IMPLANT**
KIT ZUR VERWENDUNG BEI DER IMPLANTATION EINERHERZKLAPPENPROTHESE
KIT DESTINE A ETRE UTILISE DANS LA MISE EN PLACE D'UN IMPLANT DE VALVE CARDIAQUE DE REMPLACEMENT

(30) Priority: 02.06.2021 US 202163196060 P
(43) Date of publication of application: 10.04.2024
(73) Proprietor: Boston Scientific Scimed, Inc., Maple Grove, MN 55311 (US)
(72) Inventor: O'CONNOR, Tim, Galway (IE); O'SULLIVAN, Richard, Grange East Turloughmore (IE); SHANLEY, Sean, Tuam Galway (IE)
(74) Representative: Vossius & Partner Patentanwälte Rechtsanwälte mbB
(86) International application number: PCT/US2022/031766
(87) International publication number: WO 2022/256405

(56) References cited:
- JP-A- 2021 078 858
- US-A1- 2017 209 671
- US-A1- 2019 029 790

## Description

### Cross-Reference to Related Applications

This application claims the benefit of priority of U.S. Provisional Application No. 63/196,060 filed June 2, 2021.

### Technical Field

The present disclosure pertains to medical devices and methods for manufacturing and/or using medical devices. More particularly, the present disclosure pertains to a guidewire for delivering a replacement heart valve implant.

### Background

A wide variety of intracorporeal medical devices have been developed for medical use, for example, intravascular use. Some of these devices include guidewires, catheters, medical device delivery systems (e.g., for stents, grafts, replacement valves, etc.), and the like. These devices are manufactured by any one of a variety of different manufacturing methods and may be used according to any one of a variety of methods. US 2017/209671 A1 discloses guidewires of various shapes. US 2019/029790 A1 relates to a device, system, and method for repairing heart valve function, which may include bisecting native valve leaflets for improved deployment of a prosthetic heart valve in the native valve annulus. It may include a catheter having a cutting element shaft with a cutting element configured to puncture a valve leaflet and/or make a controlled cut through the leaflet. The device may have an extendable foot configured to be positioned on an opposite side of the valve leaflet from the cutting element shaft. The device may include magnets to guide the cutting element and/or cutting element shaft in proper alignment with the extendable foot and to hold the elements in place during leaflet bisection. JP 2021 078858 A relates to a guide wire for guiding instruments including a catheter comprising a wound part and a bent part. Of the known medical devices and methods, each has certain advantages and disadvantages. There is an ongoing need to provide alternative medical devices as well as alternative methods for manufacturing and using medical devices.

### Summary

The invention is defined by the features of the independent claims.

According to an aspect of the present invention, a kit for use in a delivering a replacement heart valve implant comprises: a guidewire comprising an elongate shaft including a distal section and a proximal section extending proximally from the distal section, wherein the elongate shaft includes a coiled portion disposed within the distal section, the coiled portion being curved in a first direction within a first plane; and a forming tool configured to form a reverse curve portion in the elongate shaft proximal of the coiled portion to produce an improved guidewire, the reverse curve portion being curved in a second direction opposite the first direction; wherein the forming tool includes: a base plate; a platform secured to the base plate; a clamp configured to secure the coiled portion of the elongate shaft to the platform; at least one curved forming element configured to be secured to a post laterally offset from the platform; and a lever arm configured to rotate about the post; wherein the at least one curved forming element includes a first curved forming element having an outer surface defining a first radius.

In a first example, an improved guidewire for delivering a replacement heart valve implant comprises an elongate shaft including a distal section and a proximal section extending proximally from the distal section. The elongate shaft includes a coiled portion disposed within the distal section. The coiled portion is curved in a first direction in a first plane. The elongate shaft includes a reverse curve portion curved in a second direction opposite the first direction. The reverse curve portion is disposed proximal of the coiled portion.

In addition to any example disclosed herein, the reverse curve portion is curved in the second direction within the first plane.

In addition to any example disclosed herein, the proximal section of the elongate shaft is laterally stiffer than the distal section of the elongate shaft.

In addition to any example disclosed herein, the distal section of the elongate shaft is tapered in a distal direction.

In addition to any example disclosed herein, the elongate shaft includes a polymeric coating disposed on the coiled portion.

In addition to any example disclosed herein, the proximal section of the elongate shaft is devoid of the polymeric coating.

In addition to any example disclosed herein, the reverse curve portion is at least partially disposed in the proximal section of the elongate shaft.

In addition to any example disclosed herein, the reverse curve portion extends from a distal portion of the proximal section into the distal section.

In addition to any example disclosed herein, the at least one curved forming element includes a second curved forming element having an outer surface defining a second radius, the second radius being different from the first radius.

In addition to any example disclosed herein, the platform includes a recessed portion configured to receive the coiled portion of the elongate shaft.

In addition to any example disclosed herein, the clamp is configured to secure the coiled portion of the elongate shaft within the recessed portion of the platform.

In addition to any example disclosed herein, the reverse curve portion of the improved guidewire is curved in the second direction within the first plane.

In addition to any example disclosed herein, after forming the reverse curve portion of the improved guidewire, the coiled portion extends laterally on opposing sides of a second plane containing a central longitudinal axis of the proximal section of the improved guidewire, wherein the second plane is substantially perpendicular to the first plane.

In addition to any example disclosed herein, a method of treating a native heart valve of a patient's heart may comprise:
advancing a catheter through the native heart valve into a ventricle of the patient's heart;
advancing an improved guidewire out a distal end of the catheter into the ventricle of the patient's heart, wherein the improved guidewire includes an elongate shaft including a distal section and a proximal section extending proximally from the distal section;
   wherein the elongate shaft includes a coiled portion disposed in the distal section, the coiled portion being curved in a first direction in a first plane;
   wherein the elongate shaft includes a reverse curve portion curved in a second direction opposite the first direction;
   wherein the reverse curve portion is disposed proximal of the coiled portion;
positioning the coiled portion within the ventricle of the patient's heart such that a distal portion of the proximal section is spaced radially inward from an ostium of the native heart valve when the coiled portion of the elongate shaft is positioned within the ventricle of the patient's heart;
removing the catheter while maintaining the coiled portion of the elongate shaft within the ventricle of the patient's heart;
advancing a deployment device over the improved guidewire to the native heart valve;
deploying a replacement heart valve implant within the native heart valve, the replacement heart valve implant including an expandable framework and a plurality of valve leaflets disposed within the expandable framework; and
withdrawing the deployment device without contacting the expandable framework of the replacement heart valve implant.

In addition to any example disclosed herein, the reverse curve portion urges the distal portion of the proximal section of the elongate shaft toward a center of the native heart valve.

In addition to any example disclosed herein, the reverse curve portion is at least partially disposed upstream of the native heart valve.

In addition to any example disclosed herein, the reverse curve portion is curved in the second direction within the first plane.

In addition to any example disclosed herein, the proximal section of the elongate shaft is laterally stiffer than the distal section of the elongate shaft.

The above summary of some embodiments, aspects, and/or examples is not intended to describe each disclosed embodiment or every implementation of the present disclosure. The Figures, and Detailed Description, which follow, more particularly exemplify these embodiments.

### Brief Description of the Drawings

The disclosure may be more completely understood in consideration of the following detailed description in connection with the accompanying drawings, in which:
FIG. 1 schematically illustrates a prior art replacement heart valve delivery system;
FIG. 2 illustrates selected aspects of an example guidewire according to the disclosure;
FIG. 3 illustrates selected aspects of an example forming tool;
FIG. 4 illustrates selected aspects of a kit including a guidewire and the forming tool;
FIG. 5 illustrates selected aspects of the forming tool of FIGS. 3-4;
FIG. 6 illustrates selected aspects of placing the guidewire of the kit in the forming tool of the kit;
FIG. 7 illustrates selected aspects of forming the guidewire using the forming tool; and
FIGS. 8-13 illustrate aspects of a method of delivering a replacement heart valve implant using an example guidewire of the disclosure.

While aspects of the disclosure are amenable to various modifications and alternative forms, specifics thereof have been shown by way of example in the drawings and will be described in detail. It should be understood, however, that the intention is not to limit aspects of the disclosure to the particular embodiments described. On the contrary, the intention is to cover all modifications falling within the scope of the claims.

### Detailed Description

The following description should be read with reference to the drawings, which are not necessarily to scale, wherein like reference numerals indicate like elements throughout the several views. The detailed description and drawings are intended to illustrate but not limit the present disclosure. Those skilled in the art will recognize that the various elements described and/or shown may be arranged in various combinations and configurations without departing from the scope of the disclosure. The detailed description and drawings illustrate example embodiments of the disclosure. However, in the interest of clarity and ease of understanding, while every feature and/or element may not be shown in each drawing, the feature(s) and/or element(s) may be understood to be present regardless, unless otherwise specified.

For the following defined terms, these definitions shall be applied, unless a different definition is given in the claims or elsewhere in this specification.

All numeric values are herein assumed to be modified by the term "about," whether or not explicitly indicated. The term "about", in the context of numeric values, generally refers to a range of numbers that one of skill in the art would consider equivalent to the recited value (e.g., having the same function or result). **In** many instances, the term "about" may include numbers that are rounded to the nearest significant figure. Other uses of the term "about" (e.g., in a context other than numeric values) may be assumed to have their ordinary and customary definition(s), as understood from and consistent with the context of the specification, unless otherwise specified.

The recitation of numerical ranges by endpoints includes all numbers within that range, including the endpoints (e.g., 1 to 5 includes 1, 1.5, 2, 2.75, 3, 3.80, 4, and 5).

Although some suitable dimensions, ranges, and/or values pertaining to various components, features and/or specifications may be disclosed, one of skill in the art, incited by the present disclosure, would understand desired dimensions, ranges, and/or values may deviate from those expressly disclosed.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise. It is to be noted that in order to facilitate understanding, certain features of the disclosure may be described in the singular, even though those features may be plural or recurring within the disclosed embodiment(s). Each instance of the features may include and/or be encompassed by the singular disclosure(s), unless expressly stated to the contrary. For simplicity and clarity purposes, not all elements of the present disclosure are necessarily shown in each figure or discussed in detail below. However, it will be understood that the following discussion may apply equally to any and/or all of the components for which there are more than one, unless explicitly stated to the contrary. Additionally, not all instances of some elements or features may be shown in each figure for clarity.

Relative terms such as "proximal", "distal", "advance", "retract", variants thereof, and the like, may be generally considered with respect to the positioning, direction, and/or operation of various elements relative to a user/operator/manipulator of a device, wherein "proximal" and "retract" indicate or refer to closer to or toward the user and "distal" and "advance" indicate or refer to farther from or away from the user. **In** some instances, the terms "proximal" and "distal" may be arbitrarily assigned in an effort to facilitate understanding of the disclosure, and such instances will be readily apparent to the skilled artisan. Other relative terms, such as "upstream", "downstream", "inflow", and "outflow" refer to a direction of fluid flow within a lumen, such as a body lumen, a blood vessel, or within a device. Still other relative terms, such as "axial", "circumferential", "longitudinal", "lateral", "radial", etc. and/or variants thereof generally refer to direction and/or orientation relative to a central longitudinal axis of the disclosed structure or device.

The term "extent" may be understood to mean a greatest measurement of a stated or identified dimension, unless the extent or dimension in question is preceded by or identified as a "minimum", which may be understood to mean a smallest measurement of the stated or identified dimension. For example, "outer extent" may be understood to mean an outer dimension, "radial extent" may be understood to mean a radial dimension, "longitudinal extent" may be understood to mean a longitudinal dimension, etc. Each instance of an "extent" may be different (e.g., axial, longitudinal, lateral, radial, circumferential, etc.) and will be apparent to the skilled person from the context of the individual usage. Generally, an "extent" may be considered a greatest possible dimension measured according to the intended usage, while a "minimum extent" may be considered a smallest possible dimension measured according to the intended usage. **In** some instances, an "extent" may generally be measured orthogonally within a plane and/or cross-section, but may be, as will be apparent from the particular context, measured differently - such as, but not limited to, angularly, radially, circumferentially (e.g., along an arc), etc.

The terms "monolithic" and "unitary" shall generally refer to an element or elements made from or consisting of a single structure or base unit/element. A monolithic and/or unitary element shall exclude structure and/or features made by assembling or otherwise joining multiple discrete structures or elements together.

The terms "transaortic valve implantation" and "transcatheter aortic valve implantation" may be used interchangeably and may each be referred to using the acronym "TAVI". The terms "transaortic valve replacement" and "transcatheter aortic valve replacement" may be used interchangeably and may each be referred to using the acronym "TAVR". The terms TAVI and TAVR may be used to refer to the same or similar procedures and in at least some embodiments, the terms TAVI and TAVR may be used interchangeably.

It is noted that references in the specification to "an embodiment", "some embodiments", "other embodiments", etc., indicate that the embodiment(s) described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it would be within the knowledge of one skilled in the art to effect the particular feature, structure, or characteristic in connection with other embodiments, whether or not explicitly described, unless clearly stated to the contrary. That is, the various individual elements described below, even if not explicitly shown in a particular combination, are nevertheless contemplated as being combinable or arrangeable with each other to form other additional embodiments or to complement and/or enrich the described embodiment(s), as would be understood by one of ordinary skill in the art.

For the purpose of clarity, certain identifying numerical nomenclature (e.g., first, second, third, fourth, etc.) may be used throughout the description and/or claims to name and/or differentiate between various described and/or claimed features. It is to be understood that the numerical nomenclature is not intended to be limiting and is exemplary only. In some embodiments, alterations of and deviations from previously used numerical nomenclature may be made in the interest of brevity and clarity. That is, a feature identified as a "first" element may later be referred to as a "second" element, a "third" element, etc. or may be omitted entirely, and/or a different feature may be referred to as the "first" element. The meaning and/or designation in each instance will be apparent to the skilled practitioner.

Diseases and/or medical conditions that impact the cardiovascular system are prevalent throughout the world. Traditionally, treatment of the cardiovascular system was often conducted by directly accessing the impacted part of the system. For example, treatment of a blockage in one or more of the coronary arteries was traditionally treated using coronary artery bypass surgery. As can be readily appreciated, such therapies are rather invasive to the patient and require significant recovery times and/or treatments. More recently, less invasive therapies have been developed, for example, where a blocked coronary artery could be accessed and treated via a percutaneous catheter (e.g., angioplasty). Such therapies have gained wide acceptance among patients and clinicians.

Some mammalian hearts (e.g., human, etc.) include four heart valves: a tricuspid valve, a pulmonary valve, an aortic valve, and a mitral valve. Some relatively common medical conditions may include or be the result of inefficiency, ineffectiveness, or complete failure of one or more of the valves within the heart. Treatment of defective heart valves poses other challenges in that the treatment often requires the repair or outright replacement of the defective valve. Such therapies may be highly invasive to the patient. Disclosed herein are medical devices and/or procedures that may be used within a portion of the cardiovascular system in order to diagnose, treat, and/or repair the system, for example during and/or in conjunction with a TAVI or TAVR procedure, or in place of a TAVI or TAVR procedure in patients not suitable for such. At least some of the medical devices and/or procedures disclosed herein may be delivered and/or performed percutaneously and, thus, may be much less invasive to the patient, although other surgical methods and approaches may also be used. The devices disclosed herein may also provide a number of additional desirable features and benefits as described in more detail below. For the purpose of this disclosure, the discussion below is directed toward the treatment of a native aortic valve and will be so described in the interest of brevity. This, however, is not intended to be limiting as the skilled person will recognize that the following discussion may also apply to a mitral valve or another heart valve with no or minimal changes to the structure and/or scope of the disclosure. Similarly, the medical devices and/or procedures disclosed herein may have applications and uses in other portions of a patient's anatomy, such as but not limited to, arteries, veins, and/or other body lumens.

The figures illustrate selected components and/or arrangements of anatomy, a guidewire, a forming tool, and/or methods of using the guidewire and/or the forming tool. It should be noted that in any given figure, some features of the anatomy, the guidewire, and/or the forming tool may not be shown, or may be shown schematically, for simplicity. Additional details regarding some of the components of the anatomy, the guidewire, and/or the forming tool may be illustrated in other figures in greater detail. Additionally, not all instances of some elements or features may be shown in each figure for clarity.

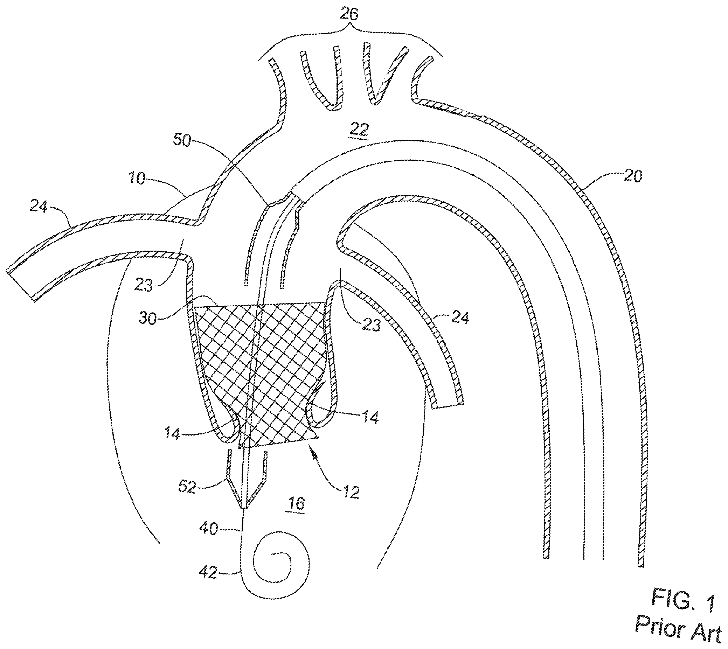

FIG. 1 illustrates a schematic partial cut-away view of a portion of a patient's heart 10 including the aortic valve 12 having valve leaflets 14, a left ventricle 16, and certain connected vasculature, such as the aorta 20 connected to the aortic valve 12 of the patient's heart 10 by the aortic arch 22, the coronary arteries 24, the ostia 23 of the coronary arteries 24, and other large arteries 26 (e.g., subclavian arteries, carotid arteries, brachiocephalic artery) that extend from the aortic arch 22 to important internal organs. As mentioned above, for the purpose of this disclosure, the discussion herein is directed toward use in treating the aortic valve 12 and will be so described in the interest of brevity. This, however, is not intended to be limiting as the skilled person will recognize that the following discussion may also apply to other heart valves, vessels, and/or treatment locations within a patient with no or minimal changes to the structure and/or scope of the disclosure.

FIG. 1 further illustrates a prior art example of treating the patient's heart 10 with a schematic example of a replacement heart valve implant 30 disposed within the aortic valve 12 of the patient's heart 10. A prior art guidewire 40 may be positioned with a distal portion 42 disposed within the left ventricle 16, and a delivery device 50 may be advanced over the prior art guidewire 40 to the aortic valve 12. The delivery device 50 is shown in a partial cutaway view. A typical prior art guidewire 40 may be configured as shown in FIG. 1, wherein proximal of the distal portion 42, a proximal portion 44 of the prior art guidewire 40 may be substantially straight. That is, while the prior art guidewire 40 may be flexible enough to navigate the patient's anatomy to the aortic valve 12, but in the absence of any constraints (e.g., vessel walls, catheter walls, tortuous anatomy, etc.), the proximal portion 44 of the prior art guidewire 40 may be substantially straight in an unstressed condition. The delivery device 50 will generally follow along the path of the proximal portion 44 of the prior art guidewire 40. Following deployment of the replacement heart valve implant 30 within the aortic valve 12, a distal portion 52 of the delivery device 50 may be offset toward an ostium of the aortic valve 12 and/or a wall of the aortic arch 22 and/or the aorta 20, as seen in FIG. 1. The natural tendency of the delivery device 50 and/or the prior art guidewire 40 to straighten may also contribute to the discussed positioning. Unfortunately, this may lead to physical contact between the distal portion 52 of the delivery device 50 and the inflow end of the replacement heart valve implant 30 as the delivery device 50 is withdrawn from the patient's heart 10. This physical contact may be undesirable because the physical contact may damage the replacement heart valve implant 30, change the positioning and/or orientation of the replacement heart valve implant 30 within the aortic valve 12, and/or may dislodge the replacement heart valve implant 30 from the aortic valve 12. Any of these results could lead to negative effects upon the patient.

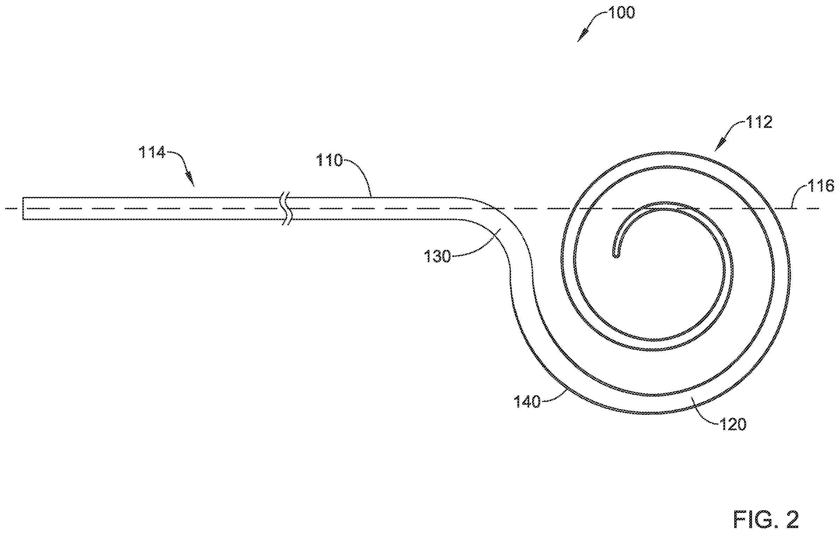

FIG. 2 illustrates selected aspects of an improved guidewire 100 for delivering a replacement heart valve implant to a native heart valve (e.g., the aortic valve 12, etc.). The improved guidewire 100 includes an elongate shaft 110 including a distal section 112 and a proximal section 114 extending proximally from the distal section 112. In some embodiments, the proximal section 114 may define a central longitudinal axis 116, which may be projected distally of the proximal section 114 for reference. The elongate shaft 110 includes a coiled portion 120 disposed within the distal section 112 in an unbiased and/or unconstrained state, wherein the coiled portion 120 of the elongate shaft 110 is curved in a first direction in a first plane, as viewed proximally to distally. In some embodiments, the coiled portion 120 may be curved in the first direction in the first plane in a distal direction along the elongate shaft 110. In some embodiments, the coiled portion 120 may extend, from proximal to distal along the length of the elongate shaft 110, in the first direction in the first plane. In some embodiments, the central longitudinal axis 116 may be disposed within the first plane. Other configurations are also contemplated. In some embodiments, the first direction may be counterclockwise. In some embodiments, the first direction may be clockwise.

The elongate shaft 110 includes a reverse curve portion 130 curved in a second direction opposite the first direction in the unbiased and/or unconstrained state, as viewed proximally to distally. In some embodiments, the reverse curve portion 130 may be disposed within and/or may be curved in the second direction within the first plane. **In** some embodiments, the reverse curve portion 130 may be curved in the second direction in the first plane in the distal direction along the elongate shaft 110. **In** some embodiments, the reverse curve portion 130 may extend, from proximal to distal along the length of the elongate shaft 110, in the second direction in the first plane. **In** some embodiments where the first direction is counterclockwise, the second direction may be clockwise. **In** some embodiments where the first direction is clockwise, the second direction may be counterclockwise. **In** at least some embodiments, the reverse curve portion 130 may be disposed proximal of the coiled portion 120 of the elongate shaft 110.

**In** some embodiments, the reverse curve portion 130 may be at least partially disposed in the proximal section 114 of the elongate shaft 110. **In** some embodiments, the reverse curve portion 130 may extend from a distal portion of the proximal section 114 of the elongate shaft 110 into the distal section 112 of the elongate shaft 110. **In** some embodiments, the reverse curve portion 130 may span a joint and/or a transition region between the proximal section 114 of the elongate shaft 110 and the distal section 112 of the elongate shaft 110. **In** some embodiments, the reverse curve portion 130 may be disposed entirely within the proximal section 114 of the elongate shaft 110.

**In** some embodiments, the coiled portion 120 of the elongate shaft 110 may extend laterally on opposing sides of a second plane containing the central longitudinal axis 116 of the proximal section 114 of the elongate shaft 110 in the unbiased and/or unconstrained state. **In** some embodiments, a first portion of the coiled portion 120 of the elongate shaft 110 may be disposed on a first side of the second plane containing the central longitudinal axis 116 of the proximal section 114 of the elongate shaft 110, and a second portion of the coiled portion 120 of the elongate shaft 110 may be disposed on a second side of the second plane containing the central longitudinal axis 116 of the proximal section 114 of the elongate shaft 110, wherein the second side of the second plane is opposite the first side of the second plane relative to the second plane. In some embodiments, the second plane may be oriented at an oblique angle to the first plane. In at least some embodiments, the second plane may be oriented substantially perpendicular to the first plane.

In some embodiments, the elongate shaft 110 may have a selected level of axial stiffness and/or pushability characteristics while also having a selected level of lateral stiffness and/or flexibility to permit navigation through the patient's vasculature. In some embodiments, the proximal section 114 of the elongate shaft 110 may be laterally stiffer than the distal section 112 of the elongate shaft 110. In some embodiments, the distal section 112 may be more flexible than the proximal section 114 of the elongate shaft 110. Other configurations are also contemplated.

In some embodiments, the distal section 112 of the elongate shaft 110 may be tapered radially inwardly in a distal direction. For example, a proximal portion of the distal section 112 of the elongate shaft may have an outer diameter that is greater than an outer diameter of a distal portion of the distal section 112. In some embodiments, the distal section 112 of the elongate shaft 110 may be tapered radially inwardly from the proximal section 114 of the elongate shaft 110 to a distalmost tip of the elongate shaft 110. In some embodiments, the distal section 112 of the elongate shaft 110 may be tapered continuously in the distal direction. In some embodiments, the distal section 112 of the elongate shaft 110 may be tapered in a stepwise fashion in the distal direction. In some embodiments, at least a portion of the coiled portion 120 may be tapered radially inwardly in the distal direction. In some embodiments, an entirety of the coiled portion 120 may be tapered radially inwardly in the distal direction.

In some embodiments, the elongate shaft 110 may include and/or may be formed from a metallic material. In some embodiments, the elongate shaft 110 is formed from stainless steel. Other configurations are also contemplated. Some suitable but non-limiting examples of materials for the elongate shaft 110 are discussed below. In some embodiments, the elongate shaft 110 may include a polymeric coating 140 disposed on the coiled portion 120 of the elongate shaft 110 and/or the distal section 112 of the elongate shaft 110. In at least some embodiments, the proximal section 114 of the elongate shaft 110 may be devoid of the polymeric coating 140. Some suitable but non-limiting examples of materials for the polymeric coating are discussed below.

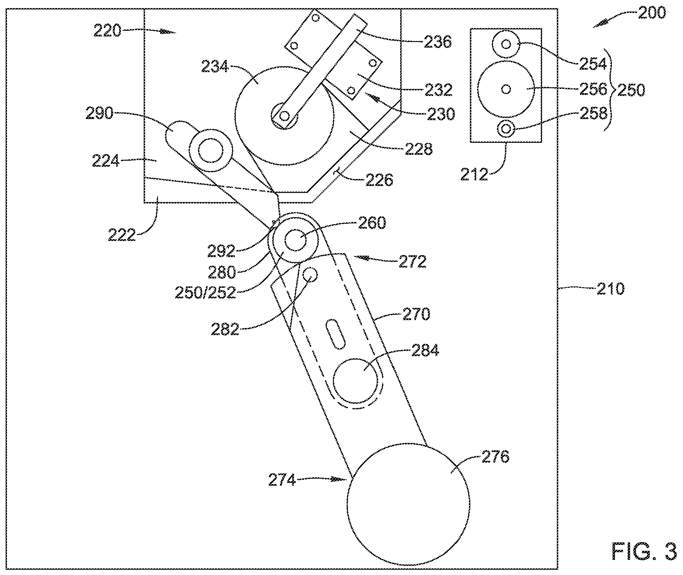

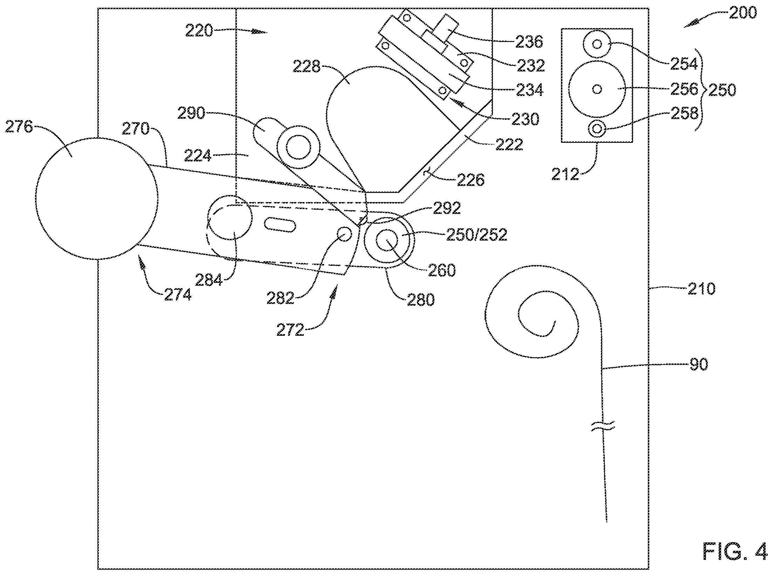

FIG. 3 illustrates selected aspects of a forming tool 200 configured to modify a guidewire 90 (e.g., FIG. 4) into the improved guidewire 100. As seen in FIG. 4, the guidewire 90 includes an elongate shaft including a distal section and a proximal section extending proximally from the distal section, wherein the distal section includes a coiled portion 92 formed in a first direction within a first plane in an unbiased and/or unconstrained state. The forming tool 200 is configured to form a reverse curve portion in the elongate shaft of the guidewire 90 proximal of the coiled portion 92 to form and/or produce the improved guidewire 100. The forming tool 200 includes a base plate 210. The forming tool 200 includes a platform 220 secured to the base plate 210. In some embodiments, the platform 220 may be removably attached to the base plate 210. In some embodiments, the platform 220 may be fixedly attached to the base plate 210. In some embodiments, the platform 220 may be attached to the base plate 210 using mechanical fasteners, adhesive bonding, welding, etc. These are only examples, and other configurations are also contemplated. Alternatively, in some embodiments, the platform 220 may be integrally formed with and/or may be monolithic with the base plate 210.

In some embodiments, the platform 220 may include a first level 222 and a second level 224. In some embodiments, the platform 220 may be stepped. In some embodiments, the platform 220 may be monolithic. In some embodiments, the first level 222 may have and/or define a first cross-sectional area, as viewed from above and/or in a top view of the forming tool 200 and/or the platform 220. In some embodiments, the second level 224 may have and/or define a second cross-sectional area, as viewed from above and/or in a top view of the forming tool 200 and/or the platform 220. In at least some embodiments, the second cross-sectional area may be less than the first cross-sectional area. In some embodiments, the second cross-sectional area may be substantially equal to the first cross-sectional area. In some embodiments, a portion of the first level 222 extending beyond and/or outside of the second level 224, as viewed from above and/or in a top view of the forming tool 200 and/or the platform 220, may define a step 226. In some embodiments, the platform 220 and/or the second level 224 of the platform 220 may include a recessed portion 228 formed therein. In some embodiments, the recessed portion 228 may be formed by removing material from a top surface of the platform 220 and/or the second level 224 of the platform 220. In some embodiments, the recessed portion 228 may be integrally formed with and/or at the same time as the top surface of the platform 220 and/or the second level 224 of the platform 220, such as by casting or injection molding, among other possible methods.

The forming tool 200 includes a clamp 230 configured to secure the coiled portion of the elongate shaft of the guidewire 90 to the platform 220, to the top surface of the platform 220 and/or the second level 224 of the platform 220, and/or within the recessed portion 228 of the platform 220 and/or the second level 224 of the platform 220. In some embodiments, the clamp 230 may include a mounting bracket 232, a clamping foot 234, and a clamping mechanism 236 extending from the mounting bracket 232 to the clamping foot 234. The mounting bracket 232 may be attached to the platform 220 and/or to the top surface of the platform 220 and/or the second level 224 of the platform 220. In some embodiments, the mounting bracket 232 may be fixedly attached to the platform 220 and/or to the top surface of the platform 220 and/or the second level 224 of the platform 220. In some embodiments, the mounting bracket 232 may be removably attached to the platform 220 and/or to the top surface of the platform 220 and/or the second level 224 of the platform 220. In some embodiments, a position of the mounting bracket 232 on the platform 220 and/or the top surface of the platform 220 and/or the second level 224 of the platform 220 may be adjustable. In some embodiments, a position of the mounting bracket 232 on the platform 220 and/or the top surface of the platform 220 and/or the second level 224 of the platform 220 may be fixed.

The clamping mechanism 236 may be one or more of a variety of suitable mechanical mechanisms for securing the clamping foot 234 and/or the coiled portion of the elongate shaft of the guidewire 90 against the platform 220, to the top surface of the platform 220 and/or the second level 224 of the platform 220, and/or within the recessed portion 228 of the platform 220 and/or the second level 224 of the platform 220. In some embodiments, the clamping mechanism 236 may include a cam mechanism, an over-center mechanism, a ratcheting mechanism, or other suitable means. These are only examples.

The forming tool 200 includes at least one curved forming element 250 configured to be secured to a post 260 laterally offset from the platform 220. The post 260 may be fixedly secured to the base plate 210. In at least some embodiments, the post 260 may be substantially cylindrical. In some embodiments, the post 260 may define a longitudinal axis oriented substantially perpendicular to the base plate 210. In some embodiments, the at least one curved forming element 250 may be disposed about the post 260. In some embodiments, the at least one curved forming element 250 may be removably secured to the post 260. The at least one curved forming element 250 may be configured to slidably engage the post 260. In some embodiments, the at least one curved forming element 250 may be rotatable with respect to the post 260. In some embodiments, the at least one curved forming element 250 may be nonrotatable with respect to the post 260. For example, the at least one curved forming element 250 and/or the post 260 may include a keying feature such as a tab and slot, a non-round shape, or other structure configured to prevent relative rotation between the at least one curved forming element 250 and the post 260. In some embodiments, the at least one curved forming element 250 may be secured to the post 260 using a screw, a bolt, a nut, a pin, or other suitable fastener or securement means capable of preventing the at least one curved forming element 250 from sliding off of the post 260.

The at least one curved forming element 250 includes a first curved forming element 252, and optionally one or more of a second curved forming element 254, a third curved forming element 256, a fourth curved forming element 258, etc. The at least one curved forming element 250 includes the first curved forming element 252 having an outer surface defining a first radius. In some embodiments, the at least one curved forming element 250 may include the second curved forming element 254 having an outer surface defining a second radius. The second radius may be different from the first radius. In some embodiments, the at least one curved forming element 250 may include the third curved forming element 256 having an outer surface defining a third radius. The third radius may be different from the first radius and the second radius. In some embodiments, the at least one curved forming element 250 may include the fourth curved forming element 258 having an outer surface defining a fourth radius. The fourth radius may be different from the first radius, the second radius, and the third radius. In some embodiments, the at least one curved forming element 250 may have a radius within a range of about 8 millimeters (mm) to about 30 mm. In some embodiments, the first radius may be about 12 mm, the second radius may be about 10.5 mm, the third radius may be about 15 mm, and the fourth radius may be about 8 mm. These are just examples. Other configurations and/or dimensions are also contemplated.

In some embodiments, the forming tool 200 may include an inventory area 212 on and/or secured to the base plate 210 for storing the at least one curved forming element 250 and/or one or more of the at least one curved forming element 250 not in use and/or removed from the post 260. In some embodiments, the inventory area 212 may include a plurality of posts configured to receive the at least one curved forming element 250 thereon. In some embodiments, the inventory area 212 may include a bin or a compartment having side walls configured to receive and/or hold the at least one curved forming element 250 therein. In some embodiments, the bin or compartment may include a removable cover. In some embodiments, the removable cover may be hingedly attached to the bin or compartment. Other configurations are also contemplated.

The forming tool 200 includes a lever arm 270 having a first end 272 and a second end 274 opposite the first end 272. The lever arm 270 is configured to rotate about the post 260 at and/or adjacent the first end 272. The lever arm 270 may define an elongate shape having a longitudinal dimension that is greater than a lateral dimension. The lever arm 270 may include a knob 276 secured to and/or disposed at the second end 274. In some embodiments, the knob 276 may be rotatable relative to the lever arm 270. In some embodiments, the knob 276 may be nonrotatable with respect to the lever arm 270.

In some embodiments, the forming tool 200 may include an attachment bar 280 secured to the lever arm 270 and the post 260. In some embodiments, the attachment bar 280 may be rotatably and/or pivotably secured to the post 260. In some embodiments, the lever arm 270 may be movably secured and/or movably attached to the lever arm 270. In some embodiments, the lever arm 270 may be pivotably attached to the attachment bar 280. In one example, the lever arm 270 may be pivotably attached to the attachment bar 280 by a pin 282 or another mechanical fastener. The pin 282 may be secured to the lever arm 270 and/or the attachment bar 280 by a press fit, a friction fit, adhesive bonding, welding, etc. Other examples of mechanical fasteners may include but are not limited to rivets, bolts, screws, etc. The attachment bar 280 may define a longitudinal dimension that is greater than a lateral dimension. The attachment bar 280 may define a longitudinal attachment bar axis extending through the post 260 and the pin 282. The lever arm 270 may define a longitudinal lever arm axis extending between the pin 282 and an axis of the knob 276.

When moving the lever arm 270 in a counterclockwise direction around the post 260, the longitudinal lever arm axis may be aligned with and/or parallel to the longitudinal attachment bar axis. In some embodiments, the lever arm 270 may include one or more protrusions extending from a bottom surface of the lever arm 270, wherein the one or more protrusions are configured to engage the attachment bar 280 when the longitudinal lever arm axis is aligned with and/or parallel to the longitudinal attachment bar axis, thus preventing any further relative rotational movement between the lever arm 270 and the attachment bar 280. Other means of preventing relative rotational movement between the lever arm 270 and the attachment bar 280 in the counterclockwise direction are also contemplated. For example, a pin, bolt, fastener, step, stop, or other feature may cause interference between the lever arm 270 and the attachment bar 280 and thus prevent relative rotational movement between the lever arm 270 and the attachment bar 280 in the counterclockwise direction. In some embodiments, a bolt or screw may extend through the lever arm 270 to engage with an attachment knob 284, wherein tightening of the attachment knob 284 secure the attachment bar 280 to the lever arm 270 and/or may tighten the attachment bar 280 against the lever arm 270 and/or increase frictional resistance to relative movement therebetween. In some embodiments, the bolt or screw may engage a slot in the lever arm 270 to prevent relative rotational movement between the lever arm 270 and the attachment bar 280 in the counterclockwise direction when the longitudinal lever arm axis is aligned with and/or parallel to the longitudinal attachment bar axis, while permitting some relative rotational movement between the lever arm 270 and the attachment bar 280 in a clockwise direction, as discussed herein with respect to operation of the forming tool 200.

In some embodiments, the lever arm 270 may include at least one detent or recess facing the attachment bar 280 and the attachment bar 280 may include at least one spring loaded feature configured to engage the at least one detent or recess when the longitudinal lever arm axis is aligned with and/or parallel to the longitudinal attachment bar axis. This engagement may be configured to prevent free relative rotational movement between the lever arm 270 and the attachment bar 280 in the clockwise direction when the lever arm 270 is rotated in the clockwise direction, except and/or until as described herein with respect to operation of the forming tool 200. **In** some embodiments, friction may prevent free relative rotational movement between the lever arm 270 and the attachment bar 280 in the clockwise direction when the lever arm 270 is rotated in the clockwise direction. Other configurations are also contemplated.

**In** some embodiments, the forming tool 200 may include a guide arm 290 removably and/or movably secured to the platform 220 and/or to the second level 224 of the platform 220. The guide arm 290 may be configured to extend over and/or may be spaced apart from the first level 222 and/or the step 226 of the platform 220. In some embodiments, the guide arm 290 may define a longitudinal dimension that is greater than a lateral dimension. In some embodiments, the guide arm 290 may be configured to move longitudinally toward and/or away from the post 260 and/or the at least one curved forming element 250 disposed on and/or secured to the post 260. Other configurations and/or movements of the guide arm 290 are also contemplated. In some embodiments, the guide arm 290 may include a distal roller 292 disposed proximate a distal end of the guide arm 290. The distal roller 292 may be configured to engage the at least one curved forming element 250 disposed on and/or secured to the post 260. At least a portion of the distal roller 292 may extend distally of the distal end of the guide arm 290. In some embodiments, the distal roller 292 may be configured to translate longitudinally relative to the guide arm 290. In at least some embodiments, the distal roller 292 may be biased in a distal direction by a spring. In some embodiments, the spring may be disposed within the guide arm 290. In some embodiments, the spring may be attached to the guide arm 290. Other configurations are also contemplated.

FIG. 4 illustrates aspects of a kit for use in delivering a replacement heart valve implant. The kit may include the forming tool 200 as described herein and a guidewire 90. The guidewire 90 may comprise an elongate shaft including a distal section and a proximal section extending proximally from the distal section, wherein the elongate shaft includes a coiled portion disposed within the distal section, the coiled portion being curved in a first direction within a first plane. The forming tool 200 may be configured to form a reverse curve portion in the elongate shaft proximal of the coiled portion to produce the improved guidewire 100 (e.g., FIG. 2), as discussed herein.

In FIG. 4, the forming tool 200 is shown in an open position. To place the forming tool 200 in the open position, the lever arm 270 and the attachment bar 280, which may be in axial alignment with each other, may be rotated and/or pivoted about the post 260 together in the clockwise direction by moving the knob 276 in the clockwise direction around the post 260 until the attachment bar 280 contacts the first level 222 of the platform 220. This contact stops rotation of the attachment bar 280. Further and/or continued rotation and/or movement of the knob 276 and/or the second end 274 of the lever arm 270 in the clockwise direction causes the lever arm 270 to pivot about the pin 282 to shift the lever arm 270 out of alignment with the attachment bar 280 such that the longitudinal lever arm axis is skewed with respect to the longitudinal attachment bar axis in the open position. The lever arm 270 may be moved over a portion of the first level 222 of the platform 220 and/or over the step 226 of the platform 220 until the lever arm 270 contacts the second level 224 of the platform 220.

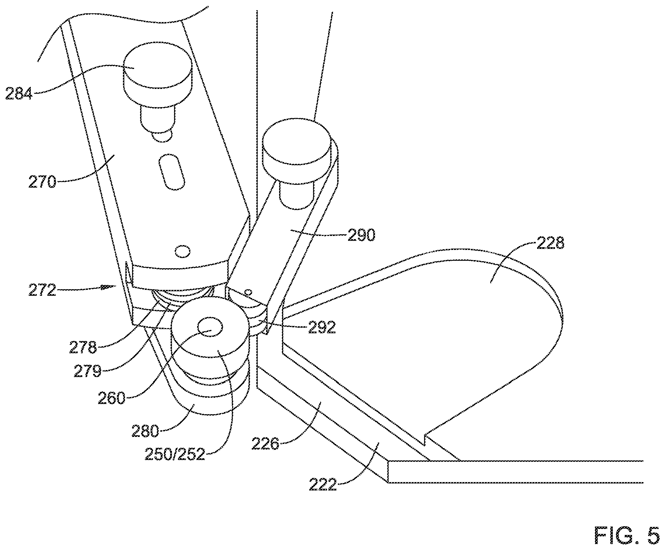

Pivoting the lever arm 270 about the pin 282 also causes a gap to open between the first end 272 of the lever arm 270 and the at least one curved forming element 250 (e.g., the first curved forming element 252, etc.) disposed on the post 260. Additionally, the guide arm 290 may extend over at least a portion of the attachment bar 280 and/or a portion of the lever arm 270. In some embodiments, the first end 272 of the lever arm 270 may include an upper arm portion and a lower arm portion. The upper arm portion may include an angled corner near and laterally offset from the pin 282, wherein a portion of the upper arm portion has been removed from the lever arm 270. When the forming tool 200 is in the open position, a side surface of the guide arm 290 may align with and/or engage against the angled corner of the upper arm portion of the lever arm 270. FIG. 5 illustrates selected aspects of the forming tool 200 in more detail.

Additionally, as seen in FIG. 5, the first end 272 of the lever arm 270 may include a roller 278 disposed between the upper arm portion and the lower arm portion. The roller 278 may have an axis of rotation that is substantially parallel to an axis of the post 260 and/or the pin 282. The roller 278 may include a groove 279 extending circumferentially around the roller 278. The groove 279 may be formed in an outer surface of the roller 278. The groove 279 may be sized and configured to receive the elongate shaft of the guidewire 90. The groove 279 may be oriented substantially perpendicular to the axis of rotation of the roller 278. In some embodiments, the roller 278 may extend beyond the first end 272 of the lever arm 270. In some embodiments, the roller 278 may be substantially flush with the first end 272 of the lever arm 270. In some embodiments, the roller 278 may be recessed within and/or from the first end 272 of the lever arm 270.

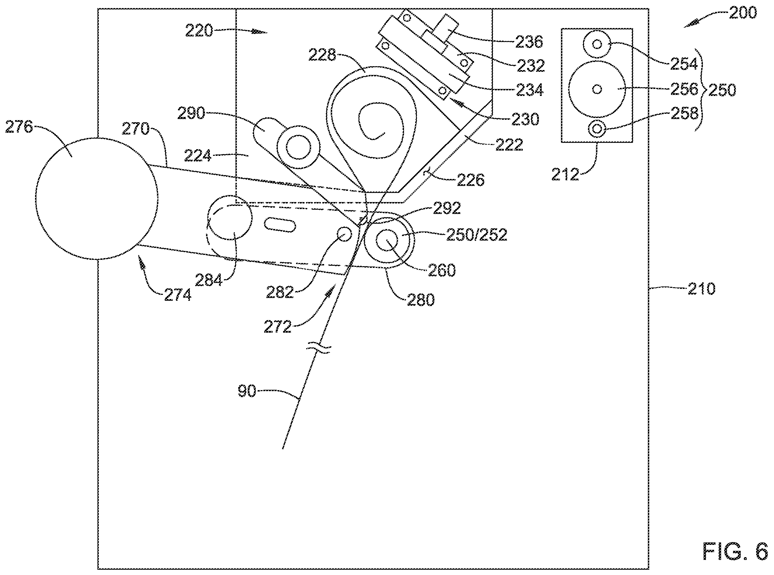

FIG. 6 illustrates aspects of a method of forming the improved guidewire 100. With the forming tool 200 in the open position, the guidewire 90 may be positioned within the forming tool 200. The coiled portion may be disposed within the recessed portion 228 of the second level 224 of the platform 220. The elongate shaft of the guidewire 90 may be positioned between the at least one curved forming element 250 (e.g., the first curved forming element 252) and the first end 272 of the lever arm 270. The elongate shaft of the guidewire 90 may be disposed between the at least one curved forming element 250 (e.g., the first curved forming element 252) and the distal roller 292 of the guide arm 290.

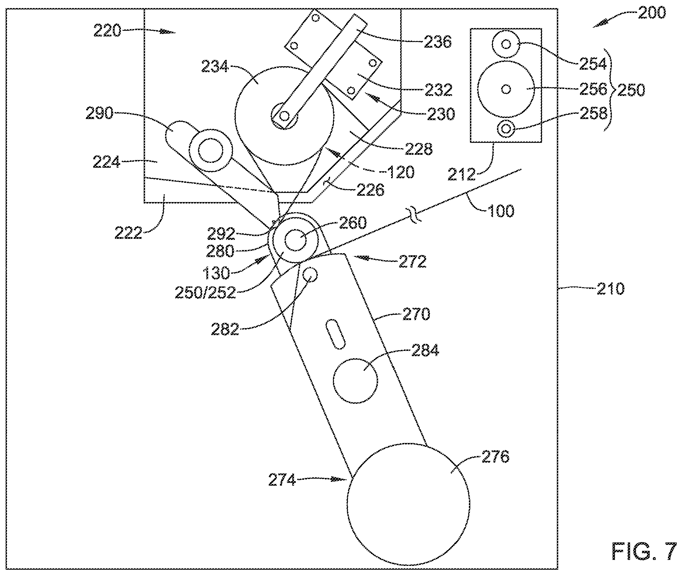

Subsequently, the clamp 230 may be closed upon the coiled portion of the guidewire 90 such that the clamping foot 234 is configured to hold, squeeze, and/or clamp the coiled portion within and/or against the recessed portion 228 of the second level 224 of the platform 220, as shown in FIG. 7. The distal roller 292 of the guide arm 290 may be configured to urge, squeeze, and/or press the guidewire 90 against the at least one curved forming element 250 (e.g., the first curved forming element 252, etc.) disposed on the post 260. In some embodiments, the guide arm 290 may be translated longitudinally toward the post 260 to engage the distal roller 292 against the guidewire 90 and/or the at least one curved forming element 250 (e.g., the first curved forming element 252, etc.) disposed on the post 260. In some embodiments, the spring of the guide arm 290 may be configured to permit the distal roller 292 to be translated longitudinally away from the post 260 without moving the guide arm 290 itself as the guidewire 90 is placed into position between the distal roller 292 and the at least one curved forming element 250 (e.g., the first curved forming element 252, etc.) disposed on the post 260, wherein releasing the spring and/or the distal roller 292 permits the distal roller 292 to translate longitudinally toward the post 260 to engage the distal roller 292 against the guidewire 90 and/or the at least one curved forming element 250 (e.g., the first curved forming element 252, etc.) disposed on the post 260.

Then, the lever arm 270 and/or the knob 276 may be rotated in the counterclockwise direction about the post 260 to bend and/or form the guidewire 90 around the at least one curved forming element 250 (e.g., the first curved forming element 252, etc.) disposed on the post 260 to form the reverse curve portion 130 of the improved guidewire 100 (e.g., to transform the guidewire 90 into the improved guidewire 100), as seen in FIG. 7. The roller 278 of the lever arm 270 and/or the distal roller 292 of the guide arm 290 may urge, squeeze, and/or press the guidewire 90 against the at least one curved forming element 250 (e.g., the first curved forming element 252, etc.) disposed on the post 260 as the lever arm 270 and/or the knob 276 is rotated in the counterclockwise direction about the post 260 to form the reverse curve portion 130 of the improved guidewire 100. Additionally, the guidewire 90 may be disposed within the groove 279 of the roller 278 to prevent the guidewire 90 from moving up or down along the at least one curved forming element 250 (e.g., the first curved forming element 252, etc.) disposed on the post 260 as the lever arm 270 and/or the knob 276 is rotated in the counterclockwise direction about the post 260 to form the reverse curve portion 130 of the improved guidewire 100 around the at least one curved forming element 250 (e.g., the first curved forming element 252, etc.) disposed on the post 260. After forming the guidewire 90 around the at least one curved forming element 250 (e.g., the first curved forming element 252, etc.) disposed on the post 260 to produce and/or form the improved guidewire 100, the reverse curve portion 130 may have a bend radius that matches and/or closely approximates the radius of the at least one curved forming element 250 (e.g., the first curved forming element 252, etc.) disposed on the post 260. As such, changing the at least one curved forming element 250 (e.g., the first curved forming element 252, etc.) disposed on the post 260 to use a different curved forming element (e.g., the second curved forming element 254, the third curved forming element 256, etc.) permits different bend radii to be applied to the guidewire 90 when forming the improved guidewire 100 and/or the reverse curve portion 130 according to a particular need or patient characteristic.

As seen in FIG. 7, the roller 278 and/or the first end 272 of the lever arm 270 may be configured to engage the at least one curved forming element 250 (e.g., the first curved forming element 252, etc.) disposed on the post 260 as the lever arm 270 and the attachment bar 280 are rotated counterclockwise about the post 260. When rotating the lever arm 270 and the attachment bar 280 counterclockwise about the post 260, the guidewire 90 may be disposed within the groove 279 of the roller 278 to prevent vertical movement of the guidewire 90 along the at least one curved forming element 250 (e.g., the first curved forming element 252, etc.) disposed on the post 260. This may also help to ensure that the reverse curve portion 130 is formed in the first plane along with the coiled portion 120. Other configurations are also contemplated.

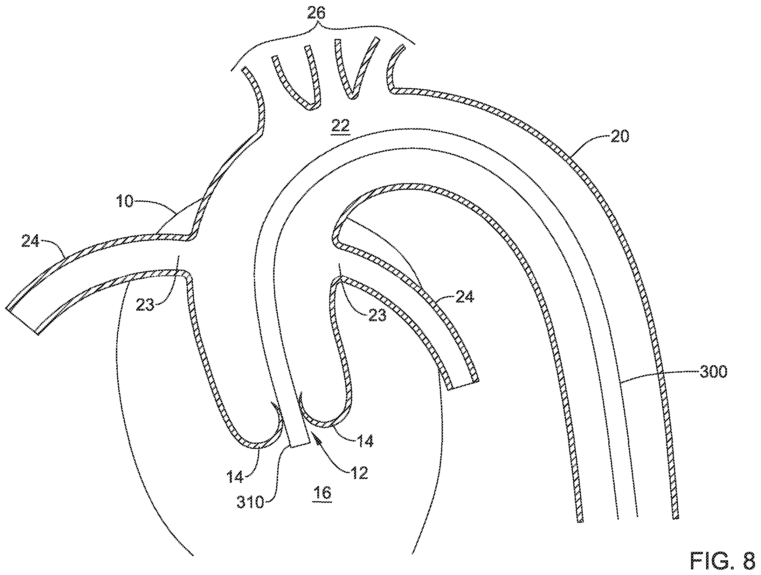

FIGS. 8-13 illustrate aspects of a method of treating a native heart valve (e.g., the aortic valve 12) of a patient's heart 10. As seen in FIG. 8, the method may include advancing a distal end 310 of a catheter 300 through the patient's vasculature (e.g., the aorta 20, etc.) to and/or through the native heart valve (e.g., the aortic valve 12) into the ventricle 16 of the patient's heart 10. The distal end 310 of the catheter 300 may be disposed within the ventricle 16 of the patient's heart 10. The catheter 300 may include one or more lumens extending through the catheter 300 to the distal end 310. At least one of the one or more lumens may be a guidewire lumen and/or a working lumen. **In** some embodiments, the patient's heart 10 may be accessed more directly and navigation through the patient's vasculature (e.g., the aorta 20) may not be necessary.

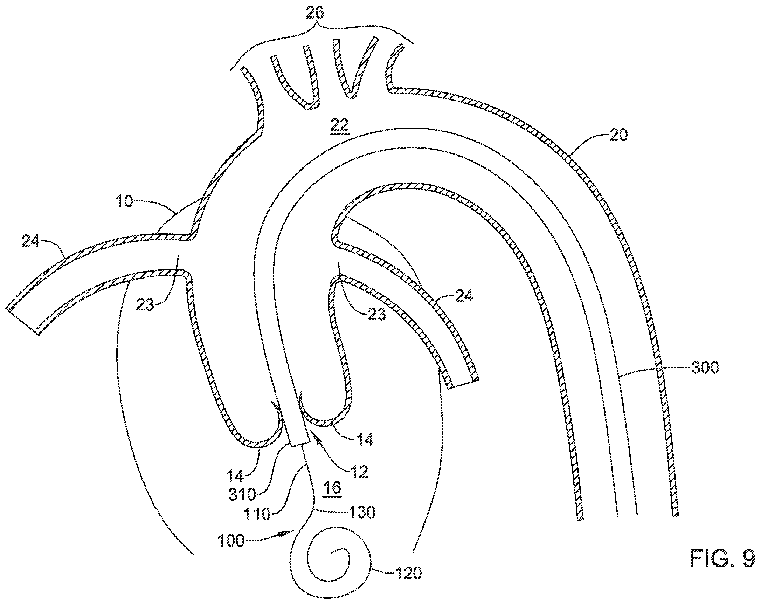

The method may include advancing the improved guidewire 100 out the distal end 310 of the catheter 300 into the ventricle 16 of the patient's heart 10, as seen in FIG. 9. The improved guidewire 100 may be configured as described herein. In some embodiments, the improved guidewire 100 may be contained and/or constrained in a straightened configuration within the one or more lumens of the catheter 300 as the catheter 300 is advancing to the native heart valve (e.g., the aortic valve 12) of the patient's heart 10. In some embodiments, the improved guidewire 100 may be advanced simultaneously with and/or within the catheter 300 through the patient's vasculature to the native heart valve (e.g., the aortic valve 12) of the patient's heart 10. In some embodiments, the improved guidewire 100 may be advanced through the catheter 300 after positioning the distal end 310 of the catheter 300 within the ventricle 16 of the patient's heart 10.

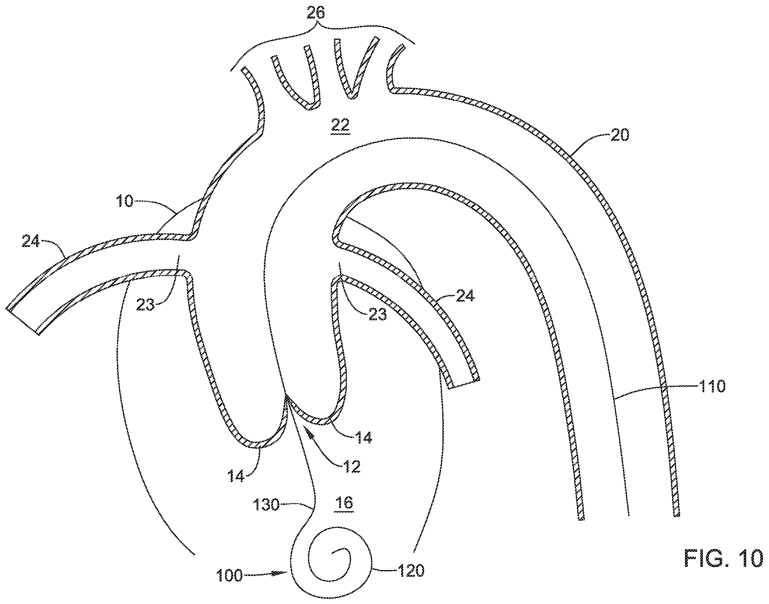

The method may include positioning the coiled portion 120 of the improved guidewire 100 within the ventricle 16 of the patient's heart 10 such that the distal portion of the proximal section 114 is spaced radially inward from an ostium of the native heart valve (e.g., the aortic valve 12) when the coiled portion 120 of the elongate shaft 110 of the improved guidewire 100 is positioned within the ventricle 16 of the patient's heart 10. The method may include removing the catheter 300 while maintaining the coiled portion 120 of the improved guidewire 100 within the ventricle 16 of the patient's heart 10, as shown in FIG. 10. FIG. 10 also illustrates the elongate shaft 110 spaced radially inwardly from the ostium of the native heart valve (e.g., the aortic valve 12) when the coiled portion 120 of the elongate shaft 110 of the improved guidewire 100 is positioned within the ventricle 16 of the patient's heart 10. The reverse curve portion 130 urges the distal portion of the proximal section 114 of the elongate shaft 110 toward a center of the native heart valve (e.g., the aortic valve 12), compared to the prior art guidewire of FIG. 1.

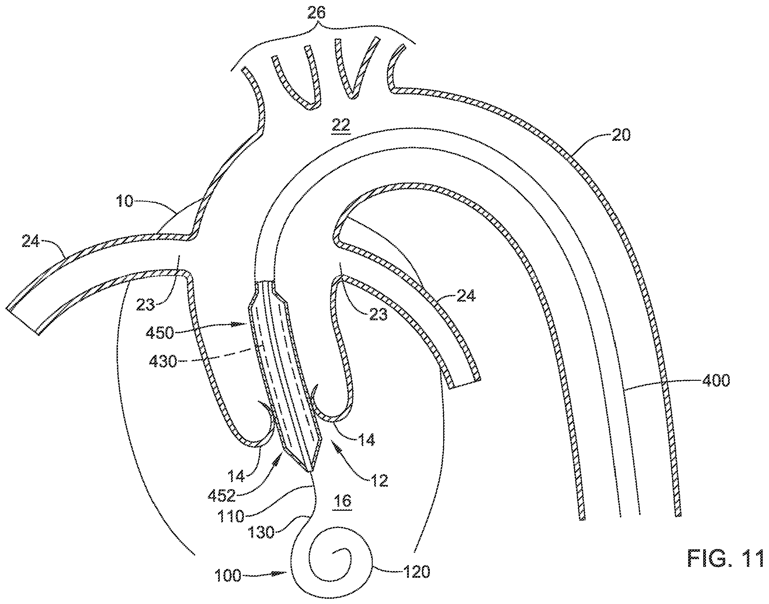

The method may include advancing a deployment device 400 over the improved guidewire 100 to the native heart valve (e.g., the aortic valve 12) of the patient's heart 10, as seen in FIG. 11. The deployment device 400 is shown in a partial cutaway view. In some embodiments, the deployment device 400 may include an outer sheath and an inner shaft axially translatable relative to the outer sheath. In some embodiments, the inner shaft of the deployment device 400 may include a central guidewire lumen extending therethrough to a distal end of the deployment device 400.

The deployment device 400 and/or the outer sheath may include a distal containment section having a replacement heart valve implant 430 disposed therein in a constrained and/or collapsed configuration. The distal containment section may include a proximal portion 450 and a distal portion 452. In some embodiments, the proximal portion 450 and the distal portion 452 of the distal containment section may be configured to axially translate relative to each other to open the distal containment section and release the replacement heart valve implant 430. In some embodiments, the proximal portion 450 of the distal containment section may be fixedly attached to and/or integrally formed with the outer sheath. In some embodiments, the distal portion 452 of the distal containment section may be fixedly attached to and/or integrally formed with the inner shaft. Therefore, relative axial translation between the outer sheath and the inner shaft may cause corresponding relative axial translation of the proximal portion 450 of the distal containment section and the distal portion 452 of the distal containment section to open the distal containment section and release the replacement heart valve implant 430. The distal containment section of the deployment device 400 may be positioned adjacent to and/or within the native heart valve (e.g., the aortic valve 12) prior to opening the distal containment section of the deployment device 400. In at least some embodiments, the distal portion 452 of the distal containment section may be disposed at least partially upstream of the native heart valve (e.g., the aortic valve 12) and/or at least partially within the ventricle 16 of the patient's heart 10 prior to opening the distal containment section of the deployment device 400.

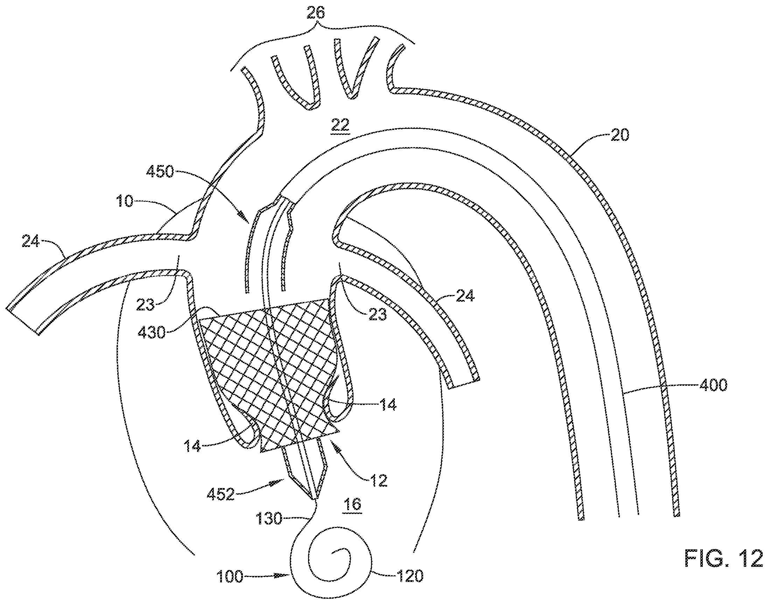

The method may include deploying the replacement heart valve implant 430 within the native heart valve (e.g., the aortic valve 12) by opening the distal containment section of the deployment device 400 while the distal containment section is disposed within the native heart valve (e.g., the aortic valve 12), as seen in FIG. 12. The replacement heart valve implant 430 may include an expandable framework and a plurality of valve leaflets disposed within the expandable framework. In some embodiments, the expandable framework may be self-expanding. In some embodiments, the expandable framework may be mechanically and/or balloon expandable. Other configurations are also contemplated.

After opening the distal containment section, the distal portion 452 of the distal containment section may be disposed upstream of the native heart valve (e.g., the aortic valve 12) and/or within the ventricle 16 of the patient's heart 10 and the proximal portion 450 of the distal containment section may be disposed downstream of the native heart valve (e.g., the aortic valve 12) and/or within the aortic arch 22 and/or the aorta 20 of the patient. The reverse curve portion 130 of the elongate shaft 110 of the improved guidewire 100 may be at least partially disposed distal and/or upstream of the native heart valve (e.g., the aortic valve 12) and/or within the ventricle 16 of the patient's heart 10. Upon opening the distal containment section, the replacement heart valve implant 430 may shift toward and/or to an expanded deployed configuration. In some embodiments, the replacement heart valve implant 430 may be released from the distal containment section and/or the deployment device 400 prior to shifting to the expanded deployed configuration. In some embodiments, the replacement heart valve implant 430 may be released from the distal containment section and/or the deployment device 400 after shifting to the expanded deployed configuration.

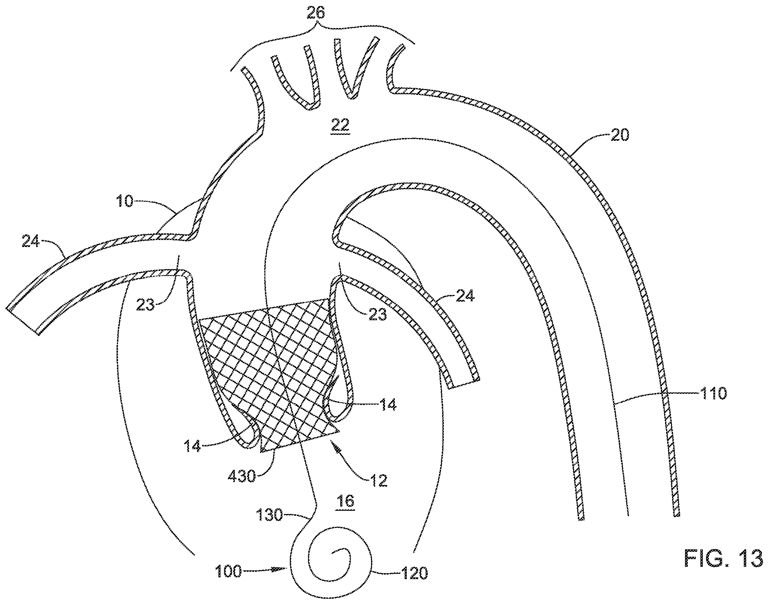

As can be seen in FIG. 12, the reverse curve portion 130 of the elongate shaft 110 of the improved guidewire 100 urges the distal portion of the proximal section 114 (not shown) of the elongate shaft 110 of the improved guidewire 100 toward a center of the native heart valve (e.g., the aortic valve 12) and/or away from the ostium of the native heart valve (e.g., the aortic valve 12) . This positioning may prevent the distal portion 452 of the distal containment section of the deployment device 400 from contacting the replacement heart valve implant 430 as the deployment device 400 is removed from the patient's heart 10, as seen in FIG. 13.

In some embodiments, the distal containment section may be closed prior to removing the deployment device 400 from the patient's heart 10 and/or vasculature. In some embodiments, the deployment device 400 may be disposed within and/or may be retracted into a delivery catheter (not shown) prior to removal from the patient's heart 10 and/or vasculature. Other configurations are also contemplated.

In some embodiments, the catheter 300, the deployment device 400, the distal containment section, and/or elements thereof may include at least one radiopaque marker for visualization during delivery and/or navigation through the patient's vasculature. The at least one radiopaque marker may permit accurate placement under fluoroscopy of the distal end 310 of the catheter 300 and/or the distal containment section of the deployment device 400 with respect to the native heart valve (e.g., the aortic valve 12), the ventricle 16, and/or the patient's heart 10.

The materials that can be used for the various components of the guidewire, the catheter, the replacement heart valve implant, the deployment device, and/or the forming tool (and/or other elements disclosed herein) and the various components thereof disclosed herein may include those commonly associated with medical devices. For simplicity purposes, the following discussion makes reference to the medical device(s). However, this is not intended to limit the devices and methods described herein, as the discussion may be applied to other elements, members, components, or devices disclosed herein, such as, but not limited to, the elongate shaft, the outer sheath, the inner shaft, etc. and/or elements or components thereof.

In some embodiments, the medical device(s) and/or other elements disclosed herein may be made from a metal, metal alloy, polymer (some examples of which are disclosed below), a metal-polymer composite, ceramics, combinations thereof, and the like, or other suitable material. Some examples of suitable metals and metal alloys include stainless steel, such as 444V, 444L, and 314LV stainless steel; mild steel; nickel-titanium alloy such as linear-elastic and/or super-elastic nitinol; other nickel alloys such as nickel-chromium-molybdenum alloys (e.g., UNS: N06625 such as INCONEL^{®} 625, UNS: N06022 such as HASTELLOY^{®} C-22^{®}, UNS: N10276 such as HASTELLOY^{®} C276^{®}, other HASTELLOY^{®} alloys, and the like), nickel-copper alloys (e.g., UNS: N04400 such as MONEL^{®} 400, NICKELVAC^{®} 400, NICORROS^{®} 400, and the like), nickel-cobalt-chromium-molybdenum alloys (e.g., UNS: R44035 such as MP35-N^{®} and the like), nickel-molybdenum alloys (e.g., UNS: N10665 such as HASTELLOY^{®} ALLOY B2^{®}), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R44003 such as ELGILOY^{®}, PHYNOX^{®}, and the like); platinum enriched stainless steel; titanium; combinations thereof; and the like; or any other suitable material.

As alluded to herein, within the family of commercially available nickel-titanium or nitinol alloys, is a category designated "linear elastic" or "non-super-elastic" which, although may be similar in chemistry to conventional shape memory and super elastic varieties, may exhibit distinct and useful mechanical properties. Linear elastic and/or non-super-elastic nitinol may be distinguished from super elastic nitinol in that the linear elastic and/or non-super-elastic nitinol does not display a substantial "superelastic plateau" or "flag region" in its stress/strain curve like super elastic nitinol does. Instead, in the linear elastic and/or non-super-elastic nitinol, as recoverable strain increases, the stress continues to increase in a substantially linear, or a somewhat, but not necessarily entirely linear relationship until plastic deformation begins or at least in a relationship that is more linear than the super elastic plateau and/or flag region that may be seen with super elastic nitinol. Thus, for the purposes of this disclosure linear elastic and/or non-super-elastic nitinol may also be termed "substantially" linear elastic and/or non-super-elastic nitinol.

In some cases, linear elastic and/or non-super-elastic nitinol may also be distinguishable from super elastic nitinol in that linear elastic and/or non-super-elastic nitinol may accept up to about 2-5% strain while remaining substantially elastic (e.g., before plastically deforming) whereas super elastic nitinol may accept up to about 8% strain before plastically deforming. Both of these materials can be distinguished from other linear elastic materials such as stainless steel (that can also be distinguished based on its composition), which may accept only about 0.2 to 0.44 percent strain before plastically deforming.

In some embodiments, the linear elastic and/or non-super-elastic nickel-titanium alloy is an alloy that does not show any martensite/austenite phase changes that are detectable by differential scanning calorimetry (DSC) and dynamic metal thermal analysis (DMTA) analysis over a large temperature range. For example, in some embodiments, there may be no martensite/austenite phase changes detectable by DSC and DMTA analysis in the range of about -60 degrees Celsius (°C) to about 120 °C in the linear elastic and/or non-super-elastic nickel-titanium alloy. The mechanical bending properties of such material may therefore be generally inert to the effect of temperature over this very broad range of temperature. In some embodiments, the mechanical bending properties of the linear elastic and/or non-super-elastic nickel-titanium alloy at ambient or room temperature are substantially the same as the mechanical properties at body temperature, for example, in that they do not display a super-elastic plateau and/or flag region. In other words, across a broad temperature range, the linear elastic and/or non-super-elastic nickel-titanium alloy maintains its linear elastic and/or non-super-elastic characteristics and/or properties.

In some embodiments, the linear elastic and/or non-super-elastic nickel-titanium alloy may be in the range of about 50 to about 60 weight percent nickel, with the remainder being essentially titanium. In some embodiments, the composition is in the range of about 54 to about 57 weight percent nickel. One example of a suitable nickel-titanium alloy is FHP-NT alloy commercially available from Furukawa Techno Material Co. of Kanagawa, Japan. Other suitable materials may include ULTANIUM^{™} (available from Neo-Metrics) and GUM METAL^{™} (available from Toyota). In some other embodiments, a superelastic alloy, for example a superelastic nitinol can be used to achieve desired properties.

In at least some embodiments, portions or all of the medical device(s) and/or other elements disclosed herein may also be doped with, made of, or otherwise include a radiopaque material. Radiopaque materials are understood to be materials capable of producing a relatively bright image on a fluoroscopy screen or another imaging technique during a medical procedure. This relatively bright image aids a user in determining the location of the medical device(s) and/or other elements disclosed herein. Some examples of radiopaque materials can include, but are not limited to, gold, platinum, palladium, tantalum, tungsten alloy, polymer material loaded with a radiopaque filler, and the like. Additionally, other radiopaque marker bands and/or coils may also be incorporated into the design of the medical device(s) and/or other elements disclosed herein to achieve the same result.

In some embodiments, a degree of Magnetic Resonance Imaging (MRI) compatibility is imparted into the medical device(s) and/or other elements disclosed herein. For example, the medical device(s) and/or components or portions thereof may be made of a material that does not substantially distort the image and create substantial artifacts (e.g., gaps in the image). Certain ferromagnetic materials, for example, may not be suitable because they may create artifacts in an MRI image. The medical device(s) or portions thereof, may also be made from a material that the MRI machine can image. Some materials that exhibit these characteristics include, for example, tungsten, cobalt-chromium-molybdenum alloys (e.g., UNS: R44003 such as ELGILOY^{®}, PHYNOX^{®}, and the like), nickel-cobalt-chromium-molybdenum alloys (e.g., UNS: R44035 such as MP35-N^{®} and the like), nitinol, and the like, and others.

In some embodiments, the medical device(s) and/or other elements disclosed herein may be made from or include a polymer or other suitable material. Some examples of suitable polymers may include polytetrafluoroethylene (PTFE), ethylene tetrafluoroethylene (ETFE), fluorinated ethylene propylene (FEP), polyoxymethylene (POM, for example, DELRIN^{®} available from DuPont), polyether block ester, polyurethane (for example, Polyurethane 85A), polypropylene (PP), polyvinylchloride (PVC), polyether-ester (for example, ARNITEL^{®} available from DSM Engineering Plastics), ether or ester based copolymers (for example, butylene/poly(alkylene ether) phthalate and/or other polyester elastomers such as HYTREL^{®} available from DuPont), polyamide (for example, DURETHAN^{®} available from Bayer or CRISTAMID^{®} available from Elf Atochem), elastomeric polyamides, block polyamide/ethers, polyether block amide (PEBA, for example available under the trade name PEBAX^{®}), ethylene vinyl acetate copolymers (EVA), silicones, polyethylene (PE), MARLEX^{®} high-density polyethylene, MARLEX^{®} low-density polyethylene, linear low density polyethylene (for example REXELL^{®}), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR^{®}), polysulfone, nylon, nylon-12 (such as GRILAMID^{®} available from EMS American Grilon), perfluoro(propyl vinyl ether) (PFA), ethylene vinyl alcohol, polyolefin, polystyrene, epoxy, polyvinylidene chloride (PVdC), poly(styrene-b-isobutylene-b-styrene) (for example, SIBS and/or SIBS 50A), polycarbonates, ionomers, biocompatible polymers, other suitable materials, or mixtures, combinations, copolymers thereof, polymer/metal composites, and the like. In some embodiments the sheath can be blended with a liquid crystal polymer (LCP). For example, the mixture can contain up to about 6 percent LCP.

In some embodiments, the medical device(s) and/or other elements disclosed herein may include a fabric material disposed over or within the structure. The fabric material may be composed of a biocompatible material, such a polymeric material or biomaterial, adapted to promote tissue ingrowth. In some embodiments, the fabric material may include a bioabsorbable material. Some examples of suitable fabric materials include, but are not limited to, polyethylene glycol (PEG), nylon, polytetrafluoroethylene (PTFE, ePTFE), a polyolefinic material such as a polyethylene, a polypropylene, polyester, polyurethane, and/or blends or combinations thereof.

In some embodiments, the medical device(s) and/or other elements disclosed herein may include and/or be formed from a textile material. Some examples of suitable textile materials may include synthetic yarns that may be flat, shaped, twisted, textured, preshrunk or un-shrunk. Synthetic biocompatible yarns suitable for use in the present disclosure include, but are not limited to, polyesters, including polyethylene terephthalate (PET) polyesters, polypropylenes, polyethylenes, polyurethanes, polyolefins, polyvinyls, polymethylacetates, polyamides, naphthalene dicarboxylene derivatives, natural silk, and polytetrafluoroethylenes. Moreover, at least one of the synthetic yarns may be a metallic yarn or a glass or ceramic yarn or fiber. Useful metallic yarns include those yarns made from or containing stainless steel, platinum, gold, titanium, tantalum or a Ni-Co-Cr-based alloy. The yarns may further include carbon, glass or ceramic fibers. Desirably, the yarns are made from thermoplastic materials including, but not limited to, polyesters, polypropylenes, polyethylenes, polyurethanes, polynaphthalenes, polytetrafluoroethylenes, and the like. The yarns may be of the multifilament, monofilament, or spun types. The type and denier of the yarn chosen may be selected in a manner which forms a biocompatible and implantable prosthesis and, more particularly, a vascular structure having desirable properties.

In some embodiments, the medical device(s) and/or other elements disclosed herein may include and/or be treated with a suitable therapeutic agent. Some examples of suitable therapeutic agents may include anti-thrombogenic agents (such as heparin, heparin derivatives, urokinase, and PPack (dextrophenylalanine proline arginine chloromethylketone)); anti-proliferative agents (such as enoxaparin, angiopeptin, monoclonal antibodies capable of blocking smooth muscle cell proliferation, hirudin, and acetylsalicylic acid); anti-inflammatory agents (such as dexamethasone, prednisolone, corticosterone, budesonide, estrogen, sulfasalazine, and mesalamine); antineoplastic/antiproliferative/anti-mitotic agents (such as paclitaxel, 5-fluorouracil, cisplatin, vinblastine, vincristine, epothilones, endostatin, angiostatin and thymidine kinase inhibitors); anesthetic agents (such as lidocaine, bupivacaine, and ropivacaine); anticoagulants (such as D-Phe-Pro-Arg chloromethyl ketone, an RGD peptide-containing compound, heparin, anti-thrombin compounds, platelet receptor antagonists, anti-thrombin antibodies, anti-platelet receptor antibodies, aspirin, prostaglandin inhibitors, platelet inhibitors, and tick antiplatelet peptides); vascular cell growth promoters (such as growth factor inhibitors, growth factor receptor antagonists, transcriptional activators, and translational promoters); vascular cell growth inhibitors (such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin); cholesterol-lowering agents; vasodilating agents; and agents which interfere with endogenous vasoactive mechanisms.

It should be understood that this disclosure is, in many respects, only illustrative. Changes may be made in details, particularly in matters of shape, size, and arrangement of steps, without exceeding the scope of the disclosure. This may include, to the extent that it is appropriate, the use of any of the features of one example embodiment being used in other embodiments. The disclosure's scope is, of course, defined in the language in which the appended claims are expressed.

## Claims

1. A kit for use in a delivering a replacement heart valve implant (430), comprising:
a guidewire (90) comprising an elongate shaft including a distal section and a proximal section extending proximally from the distal section, wherein the elongate shaft includes a coiled portion (92) disposed within the distal section, the coiled portion (92) being curved in a first direction within a first plane; and
a forming tool (200) configured to form a reverse curve portion in the elongate shaft proximal of the coiled portion (92) to produce an improved guidewire (100), the reverse curve portion being curved in a second direction opposite the first direction;
wherein the forming tool includes:
a base plate (210);
a platform (220) secured to the base plate (210);
a clamp (230) configured to secure the coiled portion (92) of the elongate shaft to the platform (220);
at least one curved forming element (250) configured to be secured to a post (260) laterally offset from the platform (220); and
a lever arm (270) configured to rotate about the post (260);
wherein the at least one curved forming element (250) includes a first curved forming element (252) having an outer surface defining a first radius.

2. The kit of claim 1, wherein the at least one curved forming element (250) includes a second curved forming element (254) having an outer surface defining a second radius, the second radius being different from the first radius.

3. The kit of any one of claims 1-2, wherein the platform (220) includes a recessed portion (228) configured to receive the coiled portion (92) of the elongate shaft.

4. The kit of claim 3, wherein the clamp (230) is configured to secure the coiled portion (92) of the elongate shaft within the recessed portion (228) of the platform (220).

5. The kit of any one of claims 1-4, wherein the reverse curve portion (130) of the improved guidewire (100) is curved in the second direction within the first plane.

6. The kit of any one of claims 1-5, wherein after forming the reverse curve portion (130) of the improved guidewire (100), the coiled portion (120) extends laterally on opposing sides of a second plane containing a central longitudinal axis (116) of the proximal section (114) of the improved guidewire (100), wherein the second plane is substantially perpendicular to the first plane.

## Patentansprüche

1. Kit zur Verwendung bei der Implantation einer Herzklappenprothese (430), das aufweist:
einen Führungsdraht (90), der einen länglichen Schaft mit einem distalen Abschnitt und einem proximalen Abschnitt aufweist, der sich proximal vom distalen Abschnitt erstreckt, wobei der längliche Schaft einen gewundenen Abschnitt (92) aufweist, der innerhalb des distalen Abschnitts angeordnet ist, wobei der gewundene Abschnitt (92) in eine erste Richtung innerhalb einer ersten Ebene gekrümmt ist; und
ein Formwerkzeug (200), das konfiguriert ist, einen Rückbiegungsabschnitt in dem länglichen Schaft proximal zum gewundenen Abschnitt (92) zu bilden, um einen verbesserten Führungsdraht (100) herzustellen, wobei der Rückbiegungsabschnitt in eine zweite Richtung entgegengesetzt zur ersten Richtung gekrümmt ist;
wobei das Formwerkzeug aufweist:
eine Grundplatte (210);
eine Plattform (220), die an der Grundplatte (210) befestigt ist;
eine Klemme (230), die konfiguriert ist, den gewundenen Abschnitt (92) des länglichen Schafts an der Plattform (220) zu befestigen;
mindestens ein gekrümmtes Formelement (250), das konfiguriert ist, an einem Pfosten (260) befestigt zu werden, der seitlich von der Plattform (220) versetzt ist; und
einen Hebelarm (270), der konfiguriert ist, sich um den Pfosten (260) zu drehen;
wobei das mindestens eine gekrümmte Formelement (250) ein erstes gekrümmtes Formelement (252) mit einer Außenfläche aufweist, die einen ersten Radius definiert.

2. Kit nach Anspruch 1, wobei das mindestens eine gekrümmte Formelement (250) ein zweites gekrümmtes Formelement (254) mit einer Außenfläche aufweist, die einen zweiten Radius definiert, wobei sich der zweite Radius vom ersten Radius unterscheidet.

3. Kit nach einem der Ansprüche 1 bis 2, wobei die Plattform (220) einen ausgesparten Abschnitt (228) aufweist, der konfiguriert ist, den gewundenen Abschnitt (92) des länglichen Schafts aufzunehmen.

4. Kit nach Anspruch 3, wobei die Klemme (230) konfiguriert ist, den gewundenen Abschnitt (92) des länglichen Schafts innerhalb des ausgesparten Abschnitts (228) der Plattform (220) zu befestigen.

5. Kit nach einem der Ansprüche 1 bis 4, wobei der Rückbiegungsabschnitt (130) des verbesserten Führungsdrahts (100) in die zweite Richtung innerhalb der ersten Ebene gekrümmt ist.

6. Kit nach einem der Ansprüche 1 bis 5, wobei nach dem Bilden des Rückbiegungsabschnitts (130) des verbesserten Führungsdrahts (100) sich der gewundene Abschnitt (120) lateral auf gegenüberliegenden Seiten einer zweiten Ebene erstreckt, die eine zentrale Längsachse (116) des proximalen Abschnitts (114) des verbesserten Führungsdrahts (100) enthält, wobei die zweite Ebene im Wesentlichen senkrecht zur ersten Ebene ist.

## Revendications

1. Kit destiné à être utilisé dans la mise en place d'un implant de valve cardiaque de remplacement (430), comprenant :
un fil-guide (90) comprenant une tige allongée comportant une section distale et une section proximale s'étendant de manière proximale à partir de la section distale, dans lequel la tige allongée comporte une partie enroulée (92) disposée à l'intérieur de la section distale, la partie enroulée (92) étant courbe dans une première direction à l'intérieur d'un premier plan ; et
un outil de formage (200) configuré pour former une partie courbe inverse dans la tige allongée à proximité de la partie enroulée (92) afin de produire un fil-guide amélioré (100), la partie courbe inverse étant courbe dans une deuxième direction opposée à la première direction ;
dans lequel l'outil de formage comporte :
une plaque de base (210) ;
une plate-forme (220) fixée à la plaque de base (210) ;
une pince (230) configurée pour fixer la partie enroulée (92) de la tige allongée à la plate-forme (220) ;
au moins un élément de formage courbe (250) configuré pour être fixé à un montant (260) décalé latéralement par rapport à la plate-forme (220) ; et
un bras de levier (270) configuré pour tourner autour du montant (260) ;
dans lequel l'au moins un élément de formage courbe (250) comporte un premier élément de formage courbe (252) ayant une surface extérieure définissant un premier rayon.

2. Kit selon la revendication 1, dans lequel l'au moins un élément de formage courbe (250) comporte un deuxième élément de formage courbe (254) ayant une surface extérieure définissant un deuxième rayon, le deuxième rayon étant différent du premier rayon.

3. Kit selon l'une des revendications 1 à 2, dans lequel la plate-forme (220) comporte une partie en retrait (228) configurée pour recevoir la partie enroulée (92) de la tige allongée.

4. Kit selon la revendication 3, dans lequel la pince (230) est configurée pour fixer la partie enroulée (92) de la tige allongée à l'intérieur de la partie en retrait (228) de la plate-forme (220).

5. Kit selon l'une des revendications 1 à 4, dans lequel la partie courbe inverse (130) du fil-guide amélioré (100) est courbe dans la deuxième direction à l'intérieur du premier plan.

6. Kit selon l'une des revendications 1 à 5, dans lequel, après avoir formé la partie courbe inverse (130) du fil-guide amélioré (100), la partie enroulée (120) s'étend latéralement sur des côtés opposés d'un deuxième plan contenant un axe longitudinal central (116) de la section proximale (114) du fil-guide amélioré (100), dans lequel le deuxième plan est sensiblement perpendiculaire au premier plan.
